# EUROPEAN PATENT APPLICATION

(11) **EP 2 128 126 A1**
(43) Date of publication of application: **02.12.2009**
(21) Application number: 08156903.0
(22) Date of filing: 26.05.2008
(51) Int. Cl.: C07C 241/02, C07C 243/40

(54) **New process for the preparation of 3-(2,2,2-Trimethylhydrazinium) propionate dihydrate**

(71) Applicant: Grindeks, a joint stock company, Riga 1057 (LV)
(72) Inventor: Zicane, Daina, LV-1048, Riga (LV); Turks, Maris, LV-1048, Riga (LV)

(57) **Abstract**

The invention provides an improved, efficient method for preparing 3-(2,2,2-trimethylhydrazinium)propionate dihydrate from 3-(2,2,2-trimethylhydrazinium)propionate methylsulfate.

## Description

### Technical Field

The present invention relates to an improved process for preparation of

3-(2,2,2-trimethylhydrazinium)propionate dihydrate (international non-proprietary name-"Meldonium").

### Background Art

A number of processes for the preparation of 3-(2,2,2-trimethylhydrazinium)propionate dihydrate is known.

The first process for preparation 3-(2,2,2-trimethylhydrazinium)propionate dihydrate is disclosed in WO 80/01068 A (INST ORGANICHESKOGO SINTEZA ) 29.05.1980 . The process starts with methyl 3-(2,2-dimethylhydrazino)propionate is treated with a methyl halide or dimethylsulphate to give the appropriate trimethylhydrazinium salt, which is transferred to 3-(2,2,2-trimethylhydrazinium)propionate by Amberlite IRA-400 (OH form). After crystallization from ethanol the inner salt is obtained as a dihydrate.

This process disclosed in above patent can be considered as a common process for 3-(2,2,2-trimethylhydrazinium)propionate dihydrate preparation.

The method has many disadvantages: strongly basic ion exchangers are unstable and undergo decomposition and oxidation during processing; they withstand only a limited number of regeneration cycles; large quantities or solvents, acids and bases as well as deionised water are needed to regenerate the resins; low ion exchange capacity and therefore high production costs of 3-(2,2,2-trimethylhydrazinium)propionate dihydrate by this process are typical. This process is not convenient for large scale production of 3-(2,2,2-trimethylhydrazinium)propionate dihydrate.

A standard method of alkaline hydrolysis of carbonic acid ester in case of an 3-(2,2,2-trimethylhydrazinium)propionate salt could not be successfully realised because of the problems of separation of 3-(2,2,2-trimethylhydrazinium)propionate dihydrate and the resulting inorganic salts. It is known that 3-(2,2,2-trimethylhydrazinium)propionate forms various double salts, some of them as well is disclosed in SU 978808 (INST ORGANICHESKOGO SINTEZA) 07.12.1982.

The above mentioned technical problem was tried to solve in WO 2008/028514 A (SILVA JORGE ) 13.03.2008 , which disclosed a method for producing 3-(2,2,2-trimethylhydrazinium)propionate dihydrate by hydrolyzed under acidic conditions with catalysis by HCl, sulphuric acid, phosphoric acid etc., esters of 3-(2,2,2-trimethylhydrazinium)propionate halide or methyl sulphate followed by neutralisation by an appropriate inorganic base (for example-sodium, potassium, calcium or magnesium hydroxide or another appropriate base, for example sodium, potassium, lithium or caesium carbonate or bicarbonate etc.) and the double salts thus obtained can be separated by the invented process using saturation the solution with carbon dioxide or sulphur dioxide.

Nevertheless method disclosed in WO 2008/028514 A (SILVA JORGE) 13.03.2008 allow to avoid use of electrodyalysis in preparation 3-(2,2,2-trimethylhydrazinium)propionate dihydrate, but at the same time double salts are formed and then have to follow process step for separation of double salts, in this case it is saturation the solution with carbon dioxide or sulphur dioxide.

### Disclosure of Invention

The above objective is achieved according to present invention by sequential combination of four process steps, starting with 1,1-dimethyhydrazine:

1. Reaction of 1,1-dimethylhydrazine with methyl acrylate by using antioxidant, thus preparation of crude 3-(2,2-dimethylhydrazino) methylpropionate;

2. Methylation of 3-(2,2-dimethylhydrazino)methylpropionate to produce methyl-3-(2,2,2-trimethylhydrazinium)propionate methylsulphate;

3. Hydrolysis of methyl-3-(2,2,2-trimethylhydrazinium)propionate methylsulphate to yield crude 3-(2,2,2-trimethylhydrazinium)propionate dihydrate;

4. Crystalization of crude 3-(2,2,2-trimethylhydrazinium)propionate dihydrate with mixture of ethanol/water solution or isopropanol/water.

Advantageous Effects of the Invention

The following Reaction Scheme 1 outlines the present method of preparation 3-(2,2,2-trimethylhydrazinium)propionate dihydrate. The method includes the following advantages:

1. Surprisingly we find out that at first step of preparation of 3-(2,2,2-trimethylhydrazinium)propionate dihydrate by using antioxidant, with acts as catalyzator, can reduce oxidation process of dimethylhydrazine and 3-(2,2-dimethylhydrazino) methylpropionate product, whereby preventing the possibility of impurities.
Tert-butylhydraquinone acts as weak protic acid and catalyzed first step process.

2. The main effort of present invention disclosed in the third step when crude product was hydrolyzed with calcium hydroxide in ethanolic mixture, by obtaining calcium sulphate dihydrate residue, which is poorly soluble in water/ethanol and fall quantitative into the residue. Thereby not formed complexes or double salts with methyl-3-(2,2,2-trimethylhydrazinium)propionate, what is the main problem by using correspond halides of compound methyl-3-(2,2,2-trimethylhydrazinium)propionate. Residue of calcium sulphate dihydrate is easily remove by filtration and filtrate contains crude product of 3-(2,2,2-trimethylhydrazinium)propionate dihydrate. No further electrodialysis is necessary.

3. This process is amenable to large scale production which does not require specialized equipment.

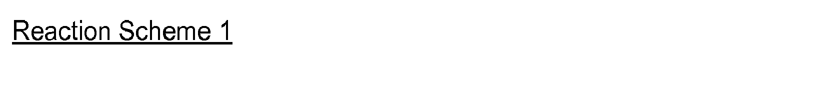

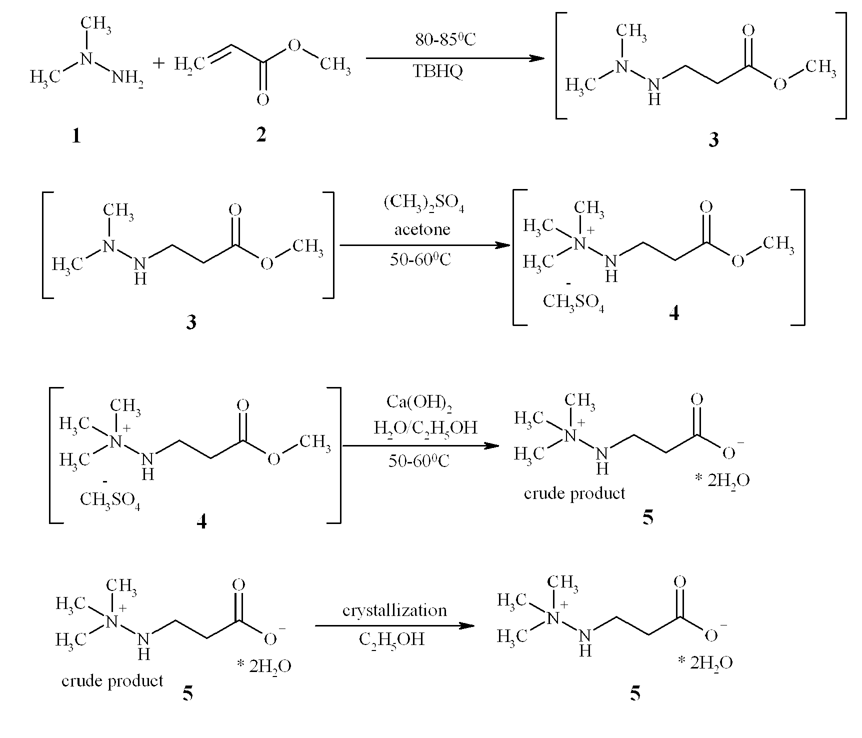

Thus, the subject matter of the present invention is a process for preparing a compound of formula 5:

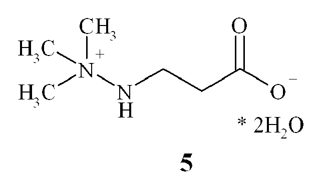

The process comprising the following steps:

a) by reaction of compound **1** with methyl acrylate of formula **2** to yield crude 3-(2,2-dimethylhydrazino)methylpropionate of formula **3** by using antioxidant, which acts as catalizator, such as tert-butylhydraquinone

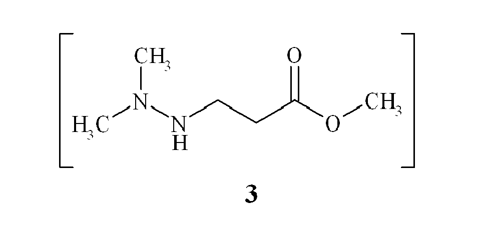

b) methylation of compound **3** with dimethylsulphate to yield crude methyl-3-(2,2,2-trimethylhydrazinium)propionate methylsulphate of formula **4**

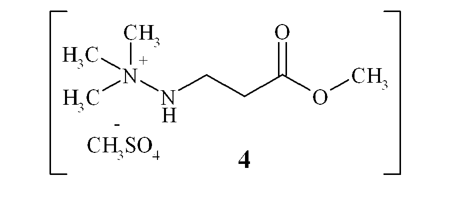

c) hydrolysis compound of formula **4** with calcium hydroxide to form crude 3-(2,2,2-trimethylhydrazinium)propionate dihydrate of formula **5**; and

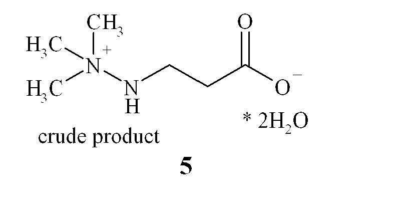

d) crystallization crude product from water/ethanol solution or water/isopropanol for obtaining the desired compound 3-(2,2,2-trimethylhydrazinium)propionate dihydrate.

### Best Mode for Carrying Out the Invention

The present invention will be described in more detail by referring to the following non-limiting examples.

### Example 1

Preparation of crude 3-(2,2-dimethylhydrazino)methylpropionate

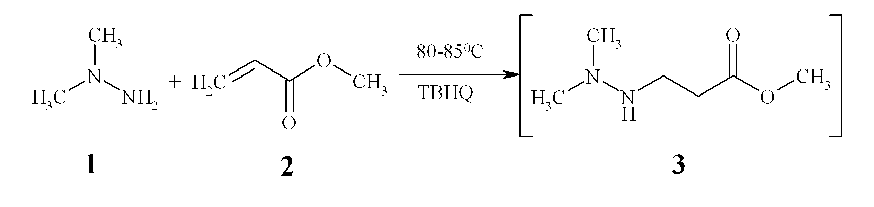

1, 1-Dimethylhydrazine (37.9g, 0.63 mol) was added to stirred tert-butylhydraquinone (0.19 g, 1.1 mmol) in 3-neck round bottom flask fitted with mechanical stirrer, a dropping funnel and a reflux condenser.

The reaction mixture was stirred and heated to 50±5°C, at which point methyl acrylate (51.6g, 0.6mol) was added. Thereafter the reaction mixture was heated at 80±85°C for 2.5 hours until formation crude 3-(2,2-dimethylhydrazino)methylpropionate (controlled by GM-MS).

### Example 2

Preparation of crude methyl-3-(2,2,2-trimethylhydrazinium)propionate methylsulphate

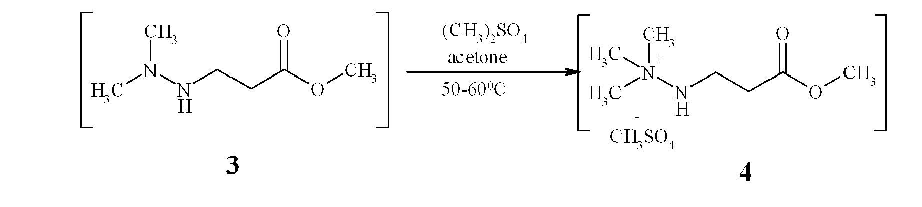

Acetone (100mL) was added to the cooled reaction mixture, which contains crude 3-(2,2-dimethylhydrazino)methylpropionate and continue cooled reaction mixture till 0-(-7)°C, at which point dimethylsulphate solution in acetone was added to the reaction mixture. The reaction mixture was heated to 50±60°C for 5 hours. The process of reaction was controlled by TLC.

The solvent was removed by distillation at a reduced pressure. The crude emulsive product of methyl-3-(2,2,2-trimethylhydrazinium)propionate methylsulphate was obtained.

### Example 3

Preparation of crude 3-(2,2,2-trimethylhydrazinium)propionate dihydrate

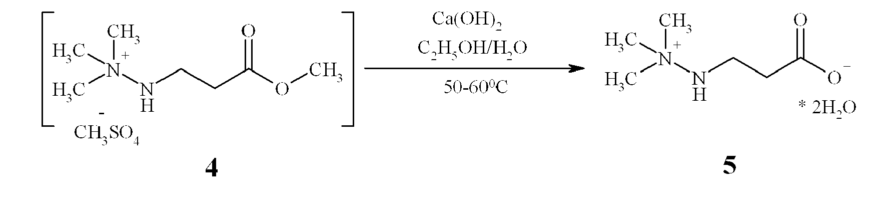

The crude emulsive methyl-3-(2,2,2-trimethylhydrazinium) propionate methylsulphate was dissolved in distilled water (500 mL) and ethanol (200mL, 96%). Calcium hydroxide (54.2g, 0.73mol) was added to stirred reaction mixture. The reaction mixture was stirred and heated to 50-60°C for 2 hours. The reaction process was controlled by TLC.

Thereafter the reaction mixture was filtered to remove calcium sulphate dihydrate. The calcium sulphate dihydrate cake on the filter was washed with ethanol (100mL).

The filtrate of reaction mixture was concentrated *in vacuo,* then isopropanol (50mL), terc-butylmethylether (20mL) and pure 3-(2,2,2-trimethylhydrazinium)propionate dihydrate as crystallization germ (0.5g) were added to reaction mixture. The obtained crystallization mixture was stirred at -15-(-5) °C. The suspension was filtered; the crude 3-(2,2,2-trimethylhydrazinium)propionate dihydrate was washed with terc-butylmethylether (150mL) on the filter.

### Example 4

Preparation of 3-(2,2,2-trimethylhydrazinium)propionate dihydrate

The crude 3-(2,2,2-trimethylhydrazinium)propionate dihydrate (162g) was added to stirred in mixture of ethanol (550mL)/water(20mL) or in mixture of isopropanol/water. The reaction mixture was heated to 70°C for 20 minutes till all crude product was dissolved, at which point it was filtered.

The filtrate was cooled to -10°C for 2 hours. The precipitate was separated by filtration. The yield 3-(2,2,2-trimethylhydrazinium)propionate dihydrate was 99.0g (90%) of white crystalline powder.

## Claims

1. A process for preparing 3-(2,2,2-trimethylhydrazinium)propionate dihydrate of formula **5** 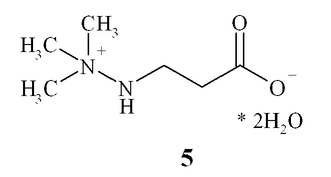 comprising hydrolysis the compound of formula **4** 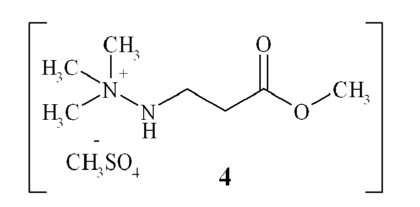 carried out with calcium hydroxide.

2. A process for preparing 3-(2,2,2-trimethylhydrazinium)propionate dihydrate of formula **5** 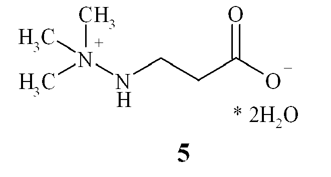 comprising the steps of: a) by reaction of compound of formula **1** with methyl acrylate in presence of antioxidant 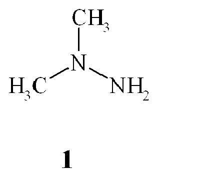 to obtain a compound of formula **3** 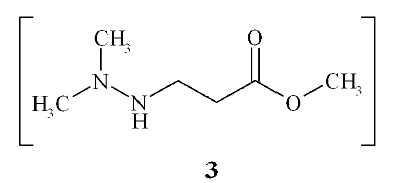
b) methylation of compound of formula **3** to obtain a compound of formula **4** 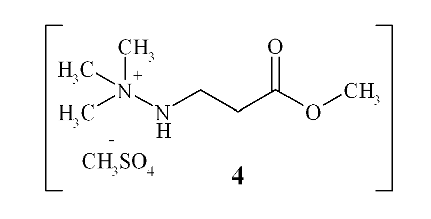
c) hydrolysis of compound of formula **4** to obtain crude compound of formula **5** 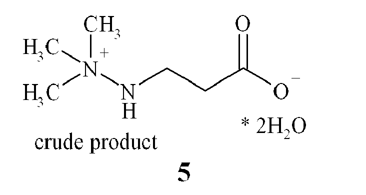
d) crystallization of crude compound of formula 5 to obtain a compound of formula **5**

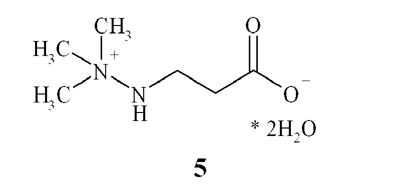

3. A process according to claim 2 wherein step a) antioxidant is tet-butylhydraquinone.

4. A process according to claim 2 wherein step b) is carried out by methylation of compound of formula 3 by dimethylsulphate.

5. A process according to claim 2 wherein step c) is hydrolysis carried out with calcium hydroxide.

6. A process according to claim 2 wherein step d) is crystallization of compound of formula 5 cariied out by water/ethanol or water/isopropanol solution.
